Europäisches Patentamt

European Patent Office

Office européen des brevets

(19)

(11) Numéro de publication : **0 004 812**
**B1**

(12) **FASCICULE DE BREVET EUROPÉEN**

(45) Date de publication du fascicule du brevet :
04.03.81

(51) Int. Cl.³ : **C 07 C 79/04**

(21) Numéro de dépôt : 79400200.6

(22) Date de dépôt : 28.03.79

(54) **Procédé de fabrication de nitroparaffines par nitration en phase gazeuse.**

(30) Priorité : 04.04.78 FR 7809837

(43) Date de publication de la demande :
17.10.79 (Bulletin 79/21)

(45) Mention de la délivrance du brevet :
04.03.81 Bulletin 81/09

(84) Etats contractants désignés :
BE CH DE FR GB IT LU NL SE

(56) Documents cités :
DE - B - 1037 438
FR - A - 2 158 708
US - A - 3 869 253

CHEMICAL ABSTRACTS
vol. 84, 19 janvier 1976, n° 3
abstract 16686y.
Columbus, Ohio, USA
I. MLADENOV et al. « Distribution of the individual nitroparaffins in the nitro compound mixture
obtained during the gas-phase nitration of pro-
pane-butane »
page 408

(73) Titulaire : **SOCIETE CHIMIQUE DE LA GRANDE
PAROISSE AZOTE ET PRODUITS CHIMIQUES**
**8, rue Cognacq-Jay**
**F-75007 Paris (FR)**

(72) Inventeur : **Lhonore, Pierre**
**1090, bd Jeanne d'Arc**
**F-59500 Douai (FR)**
Inventeur : **Quibel, Jacques**
**40, rue de la Muette**
**F-78600 Maisons Laffitte (FR)**
Inventeur : **Jacquinot, Bernard**
**59, rue Léon Bathiat**
**F-59500 Douai (FR)**

(74) Mandataire : **Bouton Neuvy, Liliane et al**
**L'Air liquide, Société Anonyme pour L'Etude et
L'Exploitation des Procédés Georges Claude 75,
Quai d'Orsay**
**F-75321 Paris Cedex 07 (FR)**

Imprimerie Jouve, 17, rue du Louvre, 75001 Paris, France

# 0 004 812

## Procédé de fabrication de nitroparaffines par nitration en phase gazeuse

La présente invention concerne un procédé de fabrication de nitroparaffines en phase gazeuse par nitration sous pression.

Par le brevet US 3869.253 on a proposé un procédé de nitration d'un hydrocarbure saturé, tel le propane, notamment en présence d'oxygène, introduit sous forme d'air, dans lequel la nitration est conduite sous une pression comprise entre 8 et 14 bars, les gaz réactants étant préchauffés sous la pression réactionnelle et introduits dans la zone réactionnelle entre 150 et 300 °C, les effluents gazeux provenant de la zone de nitration étant soumis à une trempe ou refroidissement rapide.

Ce procédé de nitration conduit à une orientation des produits finaux vers les nitropropanes avec une très nette prédominance du 2-nitropropane.

La DAS 1037.438 permet de connaître un procédé de nitration par l'acide nitrique d'un hydrocarbure tel le méthane, éthane, propane, butane, seul ou en mélange. Le mélange à nitrer peut aussi contenir un halogène, des halogénures d'alkyle ou des composés produisant des radicaux libres.

Toutefois, ces enseignements antérieurs ne donnent pas les moyens nécessaires pour modifier la structure et la distribution des nitroparaffines fabriquées.

L'un des problèmes industriels réside dans la recherche des moyens permettant dans les opérations de nitration d'obtenir un pourcentage adapté des différentes nitroparaffines en accord avec les exigences du marché. Or, il s'avère que le 2-nitropropane ne représente pas actuellement la nitroparaffine la plus valorisable. Il convient de s'adapter aux besoins de la clientèle. En outre, on recherche l'optimisation de la rentabilité de la nitration des hydrocarbures, notamment dans les installations déjà existantes et l'utilisation des matières les moins onéreuses disponibles sur le marché.

Il a été trouvé un procédé de fabrication de nitroparaffines par nitration en phase gazeuse sous pression, éventuellement en présence d'un gaz oxygéné, tel l'air, qui permet de répondre à ces nécessités.

Selon ce procédé, le réactant à nitrer est constitué par un mélange contenant du propane ; l'agent nitrant étant le peroxyde d'azote, l'acide nitrique, seuls ou en mélange ou tout agent porteur d'un groupe NO ou $NO_2$ facilement transférable ; la température et la pression réactionnelles, le temps de contact ainsi que les rapports quantitatifs entre l'agent nitrant, le mélange contenant le propane, éventuellement le gaz oxygéné, sont choisis de manière telle que la nitration soit mise en œuvre en phase gazeuse homogène.

On a découvert que la présence dans le mélange à nitrer d'une ou plusieurs nitroparaffines choisies parmi les nitroalcanes, notamment parmi les nitroparaffines fabriquées au cours de la nitration, permet de diminuer la quantité du nitroalcane produit correspondant.

L'interaction des nitroalcanes introduits en cours de réaction de nitration avec les nitroalcanes en cours de formation conduit à des modifications dans la structure des produits utilisables industriellement.

En particulier, on peut recycler les nitroparaffines en excès à un moment donné, compte tenu des besoins du marché, en vue de fabriquer les autres nitroparaffines en plus grande proportion. La quantité des nitroparaffines recyclées après séparation des effluents réactionnels peut être comprise entre 10 et 100 % de la quantité totale des nitroparaffines produites. Le recyclage est séquencé quand il concerne la totalité des nitroparaffines fabriquées, avec alternance de recyclage et production.

L'addition de 2-nitropropane au propane constitue un cas particulier en conduisant à une variation importante dans la structure des nitroalcanes correspondant à une augmentation du nitrométhane au détriment du 2-nitropropane. On constate que plus la quantité de 2-nitropropane est importante à l'entrée du réacteur, lorsqu'on a enlevé la quantité équivalente dans le liquide effluent, plus faible est le pourcentage du 2-nitropropane sortant et plus élevé est celui du nitrométhane et légèrement accrus ceux du 1-nitropropane et nitroéthane.

Le recyclage d'un des autres nitroalcanes change également la distribution des produits sortants. Il en est de même quand on associe le 2-nitropropane à une ou plusieurs autres nitroparaffines. Le recyclage de deux, voire de trois et de quatre nitroalcanes change le spectre des produits finis.

On a aussi constaté que les consommations spécifiques de peroxyde d'azote et de propane diminuent quand on recycle du 2-nitropropane ; ces consommations étant d'autant plus faibles que la quantité recyclée est plus importante. Bien que le recyclage soit une opération qui coûte de l'énergie il peut faire gagner sur la consommation des matières de départ. En outre, le prix de certaines nitroparaffines n'étant pas constant sur le marché, le choix des produits recyclés en quantités déterminées permet de trouver la rentabilité maximale de l'unité de production.

Le phénomène est du même type quelque soit l'agent nitrant.

De plus, les quantités recyclées ou introduites dans le mélange à nitrer peuvent être supérieures aux quantités produites, notamment quand on a une réserve de produits à écouler.

Les variations des chaleurs de réaction quand on recycle des alcanes nécessitent des systèmes de régulation de température, permettant soit le réchauffage du réacteur, soit son refroidissement, soit alternativement les deux.

D'autre part, on a découvert l'intérêt de la nitration de mélanges contenant du propane et un ou plusieurs autres alcanes ayant jusqu'à cinq atomes de carbone dans la molécule, tels le méthane, l'éthane, le butane et le pentane.

2

Les mélanges propane-éthane sont particulièrement avantageux. L'addition d'éthane au propane soumis à la nitration conduit au changement progressif de la distribution des nitroparaffines sortantes. L'augmentation de la quantité d'éthane dans le mélange entrant provoque une diminution du 1-nitropropane et du 2-nitropropane. La baisse de production en 2-nitropropane et 1-nitropropane est très rapide à partir d'un rapport pondéral éthane/propane de 2 à l'entrée du réacteur. Par contre, on note une très nette augmentation de la production en nitroéthane.

Ainsi, grâce à une composition choisie du mélange à nitrer en éthane par rapport au propane on a la possibilité de sortir le spectre des nitroparaffines conforme à la demande de la clientèle.

On peut envisager d'additionner au propane deux ou trois autres hydrocarbures selon la distribution de nitroparaffines recherchée.

D'autre part, toujours en fonction des produits finis attendus, il a été considéré comme intéressant de mélanger au propane au moins un des composés présents dans la phase aqueuse effluente, choisis parmi les cétones, aldéhydes, alcools, acides, nitriles formés au cours de la réaction de nitration, séparés et recyclés.

De même, suivant les cas il peut être avantageux que le mélange à nitrer contienne un composé pouvant libérer un groupement $NO_2$, tels les chloronitroparaffines contenant de 1 à 4 atomes de carbone dans la molécule.

On a aussi trouvé que la conduite de la nitration de mélanges contenant du propane, notamment des mélanges éthane-propane et propane-autres nitroparaffines en présence de gaz inertes, tels l'azote, l'oxyde de carbone et l'anhydride carbonique présente un intérêt.

Les mêmes effets ont été observés avec recyclage d'une nitroparaffine produite, tel le 2-nitropropane. Le recyclage du 2-nitropropane en présence de gaz inertes est particulièrement avantageux pour la production de nitrométhane. On constate dans ces conditions une plus grande influence de la température sur la production du 2-nitropropane.

En présence de gaz inertes quelque soit le type de mélange à base de propane il est possible d'obtenir les mêmes consommations spécifiques qu'en absence de gaz inertes à condition de choisir rigoureusement les paramètres de la réaction. Il parait avantageux de conduire la nitration des mélanges propane-éthane en présence d'au plus 50 % en volume de gaz inertes.

Il est donné ci-après des exemples qui illustrent l'invention à titre non limitatif.

## Exemple 1

### Recyclage du 2-nitropropane

On réalise une série d'essais dans lesquels la nitration du propane est mise en œuvre à une température de réaction de 340 °C, sous une pression de 10 bars, avec un temps de réaction de 7 secondes, des rapports agent nitrant, le peroxyde d'azote/propane à l'entrée en poids et air/propane dans les mêmes conditions respectivement de 0,21 et de l'ordre de 0,240.

Les résultats d'essais sont consignés dans le tableau I, dans lequel figurent les rapports en poids à l'entrée du réacteur du 2-nitropropane recyclé/propane, 2 NPr/$C_3H_8$ entrée, la composition exprimée en pourcentages en poids des nitroparaffines dans la phase liquide sortante $\varphi$ L, nitrométhane NM, nitroéthane NE, 2-nitropropane 2 NP, 1-nitropropane 1 NP, et la composition de la phase liquide sortante après prélèvement du nitropropane à recycler $\varphi$ L - 2 NP, ainsi que les consommations spécifiques du propane $C_3H_8$ c et du peroxyde d'azote $NO_2$ c consommés/nitroparaffines fabriquées en poids NP f.

Le nombre d'essais est de quatre, l'essai zéro étant l'essai comparatif (*voir tableau p. 4*).

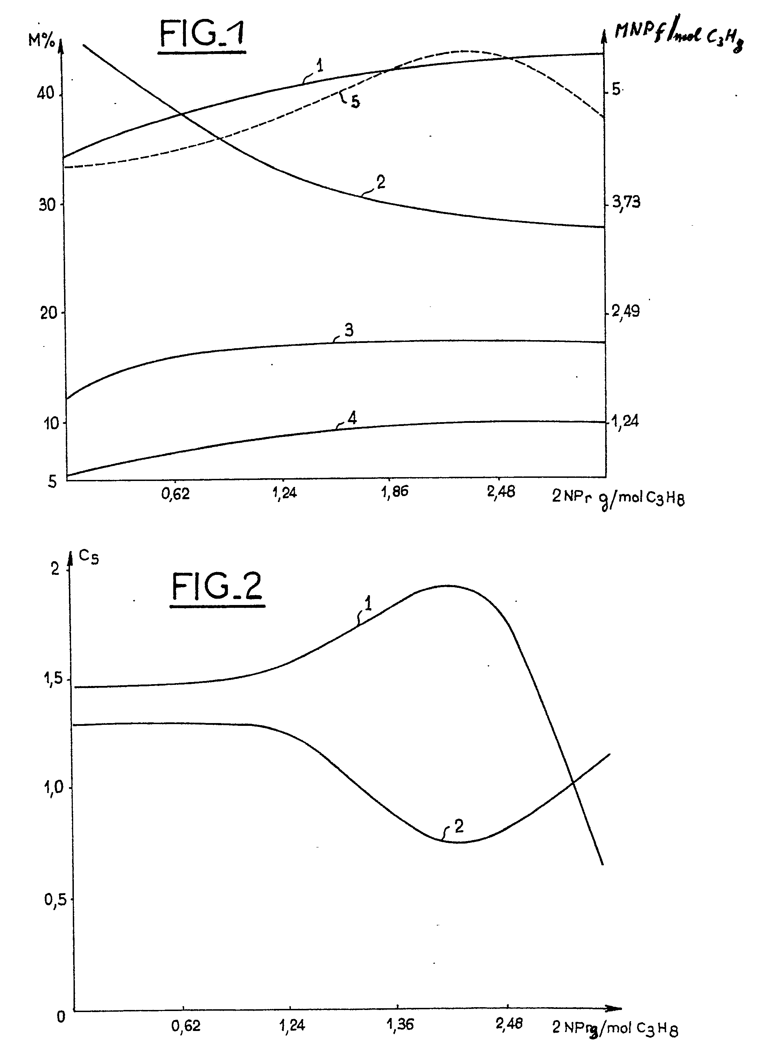

La figure 1 du dessin annexé illustre l'influence du recyclage du 2-nitropropane sur le spectre des nitroparaffines produites.

Les rapports masse en grammes de 2-nitropropane recyclée sur moles de propane introduites sont portés sur l'axe des abscisses 2 NPrg/mol $C_3H_8$. Les pourcentages en masse de chaque nitroparaffine M % sont portés sur un axe des ordonnées et les rapports masse de nitroparaffines produites sur moles de propane introduites MNPf/mol $C_3H_8$ figurent sur l'autre axe des ordonnées.

L'influence du recyclage du 2-nitropropane a été étudiée dans le cadre de la réaction de nitration conduite à 340 °C, pour un rapport propane/peroxyde d'azote de 5 moles et air/peroxyde d'azote de 1,8.

Les courbes 1, 2, 3 et 4 correspondent respectivement à la production du nitrométhane, 2-nitropropane, 1-nitropropane, nitroéthane.

La courbe 5, qui montre l'évolution de la production de l'ensemble des nitroparaffines en fonction de la quantité de 2-nitropropane recyclé, présente un maximum. A ce point, pour une même quantité de propane introduit correspond une production maximale de l'ensemble des nitroparaffines et en conséquence une taille minimale d'installation.

La figure 2 du dessin annexé est relative aux consommations spécifiques en kg par kg de l'ensemble des nitroparaffines fabriquées, dans le cadre d'une réaction de nitration du propane conduite à 340 °C, pour un rapport propane/peroxyde d'azote de 5 moles et air/peroxyde d'azote de 1,8.

Les consommations spécifiques Cs en propane et peroxyde d'azote sont portées en ordonnées et les rapports masse de 2-nitropropane recyclé/moles de $C_3H_8$ introduites 2 NPrg/mol $C_3H_8$ en abscisses. La courbe 1 correspond au propane et 2 au peroxyde d'azote.

3

TABLEAU I

| Essai n° | 0 | 1 | 2 | 3 | 4 |
|---|---|---|---|---|---|
| T °C | 340 | 340 | 340 | 340 | 340 |
| ts | 7 | 7 | 7 | 7 | 7 |
| P | 10 | 10 | 10 | 10 | 10 |
| $NO_2/C_3H_8$ entrée | 0,21 | 0,21 | 0,21 | 0,21 | 0,21 |
| Air/$C_3H_8$ entrée | 0,238 | 0,238 | 0,238 | 0,240 | 0,240 |
| 2 NPr/$C_3H_8$ entrée | 0 | 0,070 | 0,022 4 | 0,056 | 0,058 |
| Composition % φL | | | | | |
| NM | 34 | 26,6 | 32 | 29,5 | 27,5 |
| NE | 6 | 6,3 | 7,3 | 7,5 | 6,3 |
| 2 NP | 47 | 56,1 | 47 | 51 | 55,2 |
| 1 NP | 13 | 11 | 13,7 | 12 | 11 |
| Composition % φL-2 NP | | | | | |
| NM | 34 | 44 | 39,3 | 43 | 40 |
| NE | 6 | 10 | 8,7 | 11 | 9 |
| 2 NP | 47 | 28 | 35,2 | 29 | 35 |
| 1 NP | 13 | 18 | 16,8 | 17 | 16 |
| $\dfrac{C_3H_8c}{NPf}$ | 1,95 | 0,82 | 1,42 | 1,83 | 1,42 |
| $\dfrac{NO_2c}{NPf}$ | 1,27 | 1,070 | 1,28 | 0,80 | 0,70 |

De la lecture du tableau I et de la figure 1 on déduit que plus la quantité de 2-nitropropane est importante à l'entrée du réacteur, en ayant prélevé la quantité équivalente dans la phase liquide effluente, plus le pourcentage de 2-nitropropane produite est faible, tandis que celui de nitrométhane est plus élevé et que ceux de 1-nitropropane et de nitroéthane augmente légèrement. Dans ce cas, la variation importante dans la structure des nitroparaffines produites est représentée essentiellement par une augmentation du nitrométhane au détriment du 2-nitropropane.

A partir des courbes de la figure 2 on remarque que les consommations de peroxyde d'azote et de propane diminuent quand on recycle du 2-nitropropane ; ces consommations étant d'autant plus petites que la quantité de 2-nitropropanes est plus grande.

Exemple 2

Nitration de mélanges éthane-propane

On réalise une série d'essais de nitration de propane en présence d'éthane, à des températures de réaction de 320 à 340 °C, sous une pression de 10 bars, avec un temps de réaction de 5,5 secondes et un rapport agent nitrant : le peroxyde d'azote/les hydrocarbures $C_2H_6 + C_3H_8$ en entrée du réacteur (désignés par CHs) de l'ordre de 0,2.

Le nombre d'essais est de huit, l'essai zéro étant l'essai comparatif.

Les résultats d'essais sont consignés dans le tableau II, dans lequel figurent les rapports $NO_2$/CHs peroxyde d'azote sur la somme des hydrocarbures en poids à l'entrée du réacteur, de même pour l'air Air/CHs en poids, puis $C_2H_6/C_3H_8$ en poids à l'entrée du réacteur. Le tableau contient également la composition de la phase liquide en pourcentage en poids % φ L en nitrométhane NM, nitroéthane NE, 2-nitropropane 2 NP et 1-nitropropane 1 NP, ainsi que la mention des consommations spécifiques pour l'éthane, le propane, l'éthane + le propane et le peroxyde d'azote par rapport à l'ensemble des nitroparaffines produites.

TABLEAU II

| Essais n° | 0 | 1 | 2 | 3 | 4 | 5 | 6 | 7 |
|---|---|---|---|---|---|---|---|---|
| T °C | 340 | 340 | 340 | 340 | 340 | 320 | 340 | 320 |
| ts | 5,5 | 5,5 | 5,5 | 5,5 | 5,5 | 5,5 | 5,5 | 5,5 |
| P | 10 | 10 | 10 | 10 | 10 | 10 | 10 | 10 |
| $NO_2$/CHs | 0,21 | 0,20 | 0,20 | 0,20 | 0,20 | 0,20 | 0,20 | 0,20 |
| Air/CHs | 0,238 | 0,053 | 0,053 | 0,048 | 0,05 | 0,05 | 0,05 | 0,050 |
| $C_2H_6/C_3H_8$ | 0 | 1 | 1,04 | 1,02 | 2 | 2 | 4 | 4 |
| Composition % φL | | | | | | | | |
| NM | 34,5 | 29,7 | 32,8 | 31,5 | 34,0 | 27 | 31,7 | 26,5 |
| NE | 6,1 | 17,6 | 17,1 | 17,7 | 23,5 | 25,4 | 35,5 | 37,3 |
| 2 NP | 46,6 | 38 | 37,9 | 39 | 32,5 | 37,2 | 24,5 | 28,5 |
| 1 NP | 12,8 | 14,7 | 12,2 | 12,8 | 10 | 10,3 | 8,3 | 7,6 |
| $C_2H_6c$/NPf | | 0,17 | 0,57 | 0,68 | 0,60 | 0,45 | 0,132 | 0,85 |
| $C_3H_8c$/NPf | | 0,32 | 0,81 | 0,59 | 0,45 | 0,39 | 0,11 | 0,36 |
| $\dfrac{C_2H_6c + C_3H_8c}{NPf}$ | 1,95 | 0,49 | 1,38 | 1,27 | 1,05 | 0,84 | 1,43 | 1,21 |
| $NO_2c$/NPf | 1,27 | 0,9 | 0,9 | 1,18 | 1,41 | 0,89 | 1,37 | 1,12 |

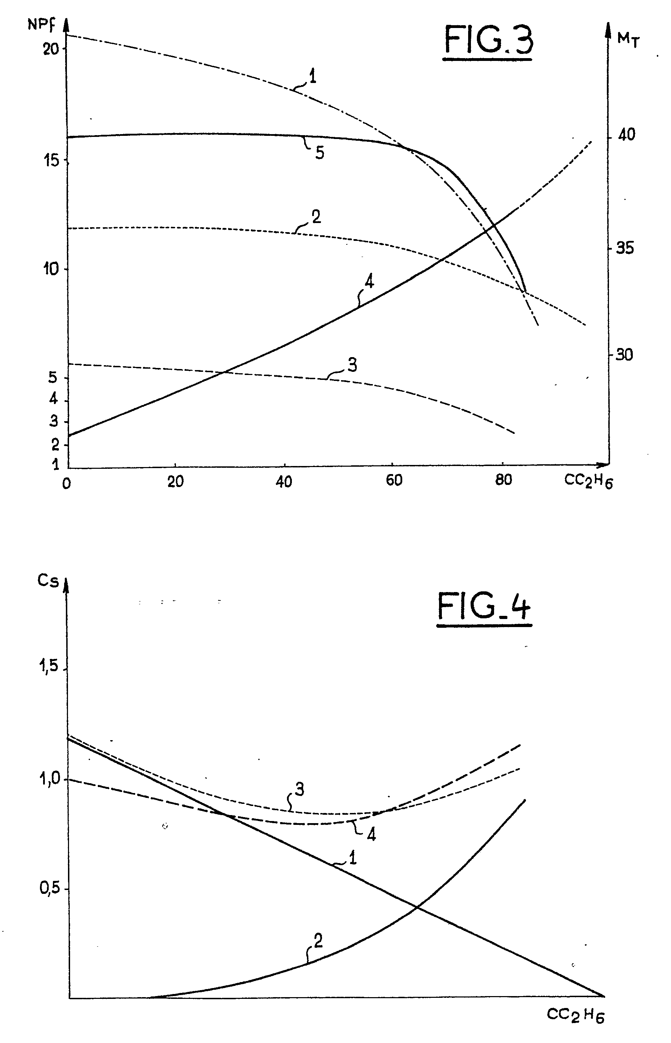

La figure 3 du dessin annexé montre l'évolution de la masse des différentes nitroparaffines produites et celle de l'ensemble de la masse en fonction du rapport éthane/propane dans le mélange à nitrer.

Les masses produites en grammes pour chaque nitroparaffine sont portées sur un axe des ordonnées NPf et la masse totale des nitroparaffines produites en grammes MT sont portées sur le second axe des ordonnées.

Les courbes 1, 2, 3 et 4 correspondent respectivement au 2-nitropropane, au nitrométhane, au 1-nitropropane et au nitroéthane. La courbe 5 correspond à l'évolution de la masse totale produite en grammes.

La figure 4 du dessin annexé représente les variations des consommations spécifiques en kg par kg de nitroparaffines produites en fonction du rapport éthane/propane à l'entrée du réacteur.

Les consommations spécifiques kg/kg Cs sont portées en ordonnées et les pourcentages des carbones introduits sous forme d'éthane C $C_2H_6$ figurent sur l'axe des abscisses.

Les courbes 1, 2, 3 et 4 correspondent respectivement aux consommations de propane, éthane, somme éthane + propane et du peroxyde d'azote.

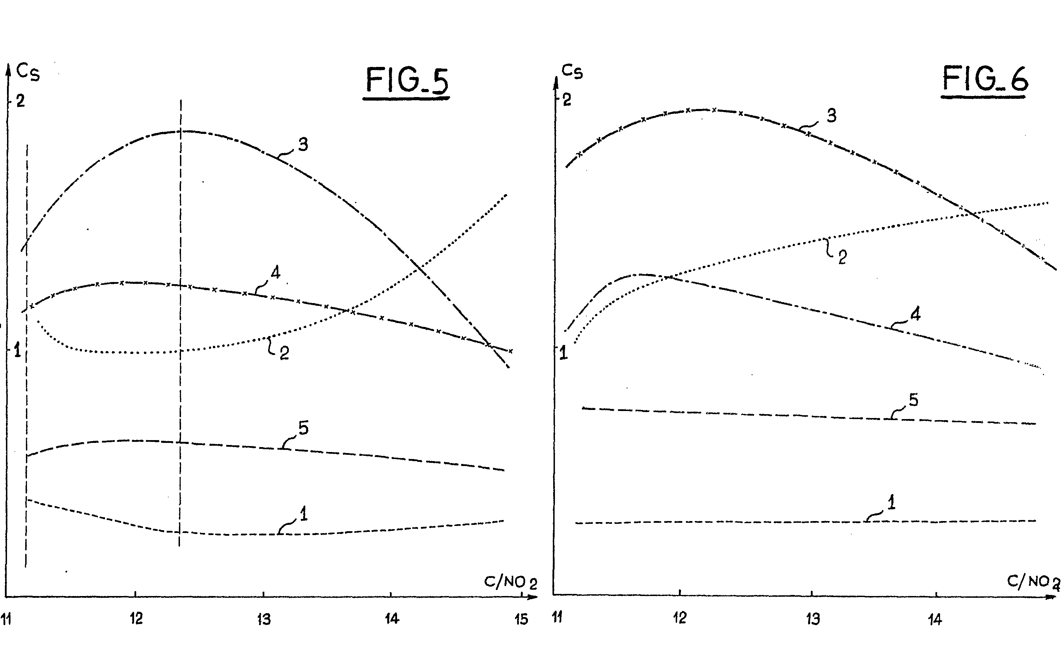

De la lecture du tableau II et à partir des courbes des figures 5 et 6 on remarque que les quantités d'éthane introduites dans le mélange à nitrer constituent un moyen de régulation du spectre des nitroparaffines produites. Quand le pourcentage d'éthane augmente dans le mélange, la quantité de 2-nitropropane diminue, ainsi que celle du 1-nitropropane. La production en 2-nitropropane s'infléchit très rapidement à partir du rapport $C_2H_6/C_3H_8$ en poids égal à 2. Par contre, on note une très nette augmentation de la production en nitroéthane. En choisissant la composition du mélange à nitrer on produit la nitroparaffine recherchée.

De plus, on note que globalement les consommations spécifiques sont moindres avec le mélange que pour le propane seul. Quand on mélange de l'éthane et du propane on obtient davantage de tous les produits finis qu'en utilisant un seul des deux hydrocarbures ; le rendement global est supérieur.

Exemple 3

Nitration de mélanges éthane-propane en présence d'inertes

On réalise une série d'essais de nitration de mélanges éthane-propane 50/50 en présence d'anhydride carbonique.

L'agent nitrant est le peroxyde d'azote. Les températures réactionnelles T °C sont comprises entre 325 et 330 °C. Les temps réactionnels ts sont compris entre 7,9 et 8,2 secondes. La pression de nitration est de 10 bars. Le $CO_2$ est présent à raison de 40 % en volume. Les rapports molaires CHs/air, c'est-à-dire somme des hydrocarbures sur air sont compris entre 49 et 67. Les proportions des hydrocarbures sont exprimées en pourcentages d'atomes grammes de carbone, c'est-à-dire que 50 % des carbones proviennent de l'éthane et 50 % du propane.

La figure 5 du dessin annexé montre l'influence du rapport C (atomes-grammes) sur $NO_2$ en moles sur la nitration d'un mélange propane-éthane en présence d'inerte $CO_2$. Les variations des consommations spécifiques Cs c'est-à-dire kg par kg de nitroparaffines produites sont portées en ordonnées et les rapports C (atome-g)/$NO_2$ (mol) $C/NO_2$ en abscisses. La courbe (1) correspond a la consommation spécifique en éthane, la courbe (2) à la consommation spécifique en propane, la courbe (3) à la consommation spécifique en peroxyde d'azote $NO_2$, la courbe (4) à la production totale en nitroparaffines, et la courbe (5) à la quantité de nitrométhane produite exprimée en grammes pour 100 atomes-grammes de carbone.

De la lecture des courbes on remarque que la consommation en $NO_2$ présente un maximum pour le rapport hydrocarbures/$NO_2$ mol 12,4 et à une température de nitration de 325 °C ; la consommation en propane étant minimale.

On réalise une autre série d'essais avec le même mélange éthane-propane (CHs) % carbones dans éthane 50 et propane 50, en présence de $CO_2$ 40 % volume, les rapports ($C/O_2$) mol étant compris entre 49 et 67, le temps réactionnel de 7,9 à 8,1 secondes, à des températures de nitration comprises entre 323 et 326 °C. On conduit la réaction en présence de 2-nitropropane recyclé (2 NPr/CHs) mol 0,009 à 0,011.

La figure 6 du dessin annexé montre l'influence du rapport C (atomes-grammes) sur $NO_2$ en moles sur la nitration de mélanges propane-éthane en présence d'inerte ($CO_2$) et de 2-nitropropane recyclé. La courbe (1) correspond à la consommation spécifique en éthane, la courbe (2) à la consommation spécifique en propane, la courbe (3) à la consommation spécifique en $NO_2$, la courbe (4) à la production totale en nitroparaffines et la courbe (5) à la quantité de nitrométhane produite exprimée en grammes pour 100 atomes-grammes de carbone. Les consommations spécifiques Cs (kg par kg de nitroparaffines produites) sont portées en ordonnées et les rapports $C/NO_2$ en abscisses.

On observe les mêmes effets que précédemment et l'intérêt du recyclage du 2-nitropropane pour la production de nitrométhane.

## Revendications

1. Procédé de fabrication de nitroparaffines par nitration en phase gazeuse homogène sous pression éventuellement en présence d'un gaz oxygéné, selon lequel le réactant à nitrer est constitué par un mélange contenant du propane, l'agent nitrant étant le peroxyde d'azote, l'acide nitrique, seul ou en mélange ou tout agent porteur d'un groupe NO ou $NO_2$ facilement transférable, caractérisé en ce que le mélange à nitrer contient en outre une ou plusieurs nitroparaffines choisies parmi les nitro-alcanes produits.

2. Procédé de fabrication de nitroparaffines selon la revendication 1, caractérisé en ce que les nitroparaffines introduites dans le mélange réactionnel sont séparées des effluents réactionnels et recyclées ; la quantité des nitroparaffines recyclées représentant 10 à 100 % de la quantité des nitroparaffines produites.

3. Procédé de fabrication de nitroparaffines selon la revendication 2, caractérisé en ce que le recyclage est séquencé quand il concerne la totalité des nitroparaffines produites, avec alternance de recyclage et production de nitroparaffines.

4. Procédé de fabrication de nitroparaffines selon la revendication 1, caractérisé en ce que la nitroparaffine présente dans le mélange à nitrer est le 2-nitropropane associé éventuellement à une ou plusieurs autres nitroparaffines.

5. Procédé de fabrication de nitroparaffines selon la revendication 1, caractérisé en ce que le mélange à nitrer contient du propane et un ou plusieurs autres alcanes ayant jusqu'à cinq atomes de carbone dans la molécule.

6. Procédé de fabrication de nitroparaffines selon la revendication 1, caractérisé en ce que l'alcane associé au propane est l'éthane.

7. Procédé de fabrication de nitroparaffines selon la revendication 1, caractérisé en ce que le mélange à nitrer contient en outre un gaz inerte tels l'azote, l'oxyde de carbone, l'anhydride carbonique.

8. Procédé de fabrication de nitroparaffines selon la revendication 1, caractérisé en ce que le mélange à nitrer contient en outre un composé pouvant libérer un groupement $NO_2$, tels les chloronitro-paraffines contenant 1 à 4 atomes de carbone dans la molécule.

## Ansprüche

1. Verfahren zur Herstellung von Nitroparaffinen durch Nitrierung in homogener Gasphase unter Druck, gegebenenfalls in Gegenwart eines sauerstoffhaltigen Gases, wobei der zu nitrierende Reaktionspartner aus einem propanhaltigen Gemisch besteht und wobei das Nitriermittel Stickstoffperoxid, Salpetersäure, allein oder im Gemisch miteinander, oder irgendein Mittel, das eine leicht übertragbare NO- oder $NO_2$-Gruppe trägt, ist, dadurch gekennzeichnet, daß das zu nitrierende Gemisch zusätzlich ein oder mehrere Nitroparaffine enthält, die unter den erzeugten Nitroalkanen ausgewählt sind.

2. Verfahren zur Herstellung von Nitroparaffinen nach Anspruch 1, dadurch gekennzeichnet, daß die in das Reaktionsgemisch eingeführten Nitroparaffine von den Reaktionsausläufen abgetrennt und rückgeführt werden, wobei die Menge der rückgeführten Nitroparaffine 10 bis 100 % der Menge der erzeugten Nitroparaffine ausmacht.

3. Verfahren zur Herstellung von Nitroparaffinen nach Anspruch 2, dadurch gekennzeichnet, daß die Rückführung, was die Gesamtheit der erzeugten Nitroparaffine betrifft, eine Sequenz mit alternierendem Wechsel von Rückführung und Produktion von Nitroparaffinen ist.

4. Verfahren zur Herstellung von Nitroparaffinen nach Anspruch 1, dadurch gekennzeichnet, daß das in dem zu nitrierenden Gemisch vorliegende Nitroparaffin 2-Nitropropan ist, das gegebenenfalls mit einem oder mehreren anderen Nitroparaffinen vereinigt ist.

5. Verfahren zur Herstellung von Nitroparaffinen nach Anspruch 1, dadurch gekennzeichnet, daß das zu nitrierende Gemisch Propan und ein oder mehrere andere Alkane mit bis zu 5 Kohlenstoffatomen im Molekül enthält.

6. Verfahren zur Herstellung von Nitroparaffinen nach Anspruch 1, dadurch gekennzeichnet, daß das mit Propan vereinigte Alkan Äthan ist.

7. Verfahren zur Herstellung von Nitroparaffinen nach Anspruch 1, dadurch gekennzeichnet, daß das zu nitrierende Gemisch zusätzlich ein Inertgas, wie Stickstoff, Kohlenmonoxid oder Kohlendioxid, enthält.

8. Verfahren zur Herstellung von Nitroparaffinen nach Anspruch 1, dadurch gekennzeichnet, daß das zu nitrierende Gemisch zusätzlich eine Verbindung enthält, die eine $NO_2$-Gruppe freisetzen kann, wie die Chlornitroparaffine mit 1 bis 4 Kohlenstoffatomen im Molekül.

**Claims**

1. Process for the production of nitro paraffins by nitration in homogeneous gaseous phase under pressure, possibly in presence of an oxygen containing gas, whereby the reactant to be nitrated consists of a mixture containing propane, the nitrating agent being nitrogen peroxide, nitric acid, solely or mixed together, or any agent having a NO or $NO_2$-group easily transferable, characterized in that the mixture to be nitrated contains additionally one or several nitro paraffins selected from the group of the produced nitro alkanes.

2. Process for the production of nitroparaffins according to claim 1, characterized in that the nitro paraffins introduced into the reaction mixture are separated from the reaction effluents and recycled, whereby the quantity of the recycled nitroparaffins represents 10 to 100 percent of the quantity of the produced nitro paraffins.

3. Process for the production of nitro paraffins according to claim 2, characterized in that concerning the totality of the produced nitro paraffins, the recycling is a sequence with alternating recycling and production of nitro paraffins.

4. Process for the production of nitro paraffins according to claim 1, characterized in that the nitro paraffin present in the mixture to be nitrated is the 2-nitropropane possibly associated with one or several other nitro paraffins.

5. Process for the production of nitro paraffins according to claim 1, characterized in that the mixture to be nitrated contains propane and one or several other alkanes having up to 5 carbon atoms in the molecule.

6. Process for the production of nitro paraffins according to claim 1, characterized in that the alkane associated with propane is ethane.

7. Process for the production of nitro paraffins according to claim 1, characterized in that the mixture to be nitrated contains additionally an inert gas, such as nitrogen, carbon monoxide or carbon dioxide.

8. Process for the production of nitro paraffins according to claim 1, characterized in that the mixture to be nitrated contains additionally a compound, which is able to release a $NO_2$-group, such as the chloro nitro paraffins containing 1 to 4 carbon atoms in the molecule.

FIG.1

FIG.2

FIG.3

FIG.4

FIG_6

FIG_5